(19) Europäisches Patentamt
European Patent Office
Office européen des brevets

(11) EP 2 316 409 A1

(12) **DEMANDE DE BREVET EUROPEEN**

(43) Date de publication:
**04.05.2011 Bulletin 2011/18**

(21) Numéro de dépôt: **10175376.2**

(22) Date de dépôt: **06.09.2010**

(51) Int Cl.:
***A61K 8/31** (2006.01)* ***A61K 8/33** (2006.01)*
***A61K 8/37** (2006.01)* ***A61K 8/81** (2006.01)*
***A61K 8/90** (2006.01)* ***A61Q 1/00** (2006.01)*
***A61Q 19/00** (2006.01)*

(84) Etats contractants désignés:
**AL AT BE BG CH CY CZ DE DK EE ES FI FR GB GR HR HU IE IS IT LI LT LU LV MC MK MT NL NO PL PT RO SE SI SK SM TR**
Etats d'extension désignés:
**BA ME RS**

(30) Priorité: **08.09.2009 FR 0956108**

(71) Demandeur: **L'Oréal**
**75008 Paris (FR)**

(72) Inventeurs:
- **Arnaud, Pascal**
  **94240, L'Hay Les Roses (FR)**
- **Thibout, Camille**
  **75020, Paris (FR)**

(74) Mandataire: **Leonard, Armelle**
**L'Oréal**
**D.I.P.I.**
**25-29, Quai Aulagnier**
**92665 Asnières-sur-Seine Cedex (FR)**

(54) **Composition cosmétique contenant un copolymère séquencé et une huile non volatile spécifique**

(57) L'invention concerne notamment une composition cosmétique de soin et/ou de maquillage des matières kératiniques comprenant au moins une phase grasse liquide et au moins :
a) un copolymère éthylénique séquence (également appelé polymère éthylénique séquencé), contenant au moins une première séquence ayant une température de transition vitreuse (Tg) supérieure ou égale à 40°C et étant issue en totalité ou en partie de un ou plusieurs premiers monomères, qui sont tels que l'homopolymère préparé à partir de ces monomères a une température de transition vitreuse supérieure ou égale à 40°C, et au moins une deuxième séquence ayant une température de transition vitreuse inférieure ou égale à 20°C et étant issue en totalité ou en partie de un ou plusieurs deuxièmes monomères, qui sont tels que l'homopolymère préparé à partir de ces monomères a une température de transition vitreuse inférieure ou égale à 20°C, ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité I supérieur à 2,
b) une huile non volatile hydrocarbonée caractérisée par un paramètre de solubilité de Hansen δa allant de 2 à 7 $(J/cm^3)^{1/2}$ et une masse moléculaire inférieure ou égale à 300g/mol, et
c) une huile volatile.

EP 2 316 409 A1

## Description

La présente invention concerne des compositions cosmétiques de soin et/ou de maquillage des matières kératiniques, en particulier sous la forme d'émulsions E/H, présentant de bonnes propriétés de tenue et avantageusement de confort après application sur les matières kératiniques, en particulier la peau.

Lorsque les femmes utilisent un produit cosmétique tel qu'un produit de maquillage, un produit solaire, ou encore un produit de soin, elles souhaitent que ce produit présente, après application, une bonne tenue.

Il est connu de l'homme de l'art d'utiliser des polymères pour obtenir ces propriétés de tenue au cours de la journée.

Ces polymères sont de natures chimiques très différentes et sont véhiculés soit dans une phase grasse soit dans une phase aqueuse.

On peut citer à titre d'exemples les résines de silicones, les polyacrylates, les latex etc.

Ces polymères sont souvent associés à des composés volatils qui permettent d'améliorer la tenue et des huiles non volatiles qui favorisent le confort.

Toutefois, si ces huiles non volatiles ne sont pas choisies convenablement vis-à-vis du polymère, elles peuvent être préjudiciables aux propriétés de tenue de la composition et même de son confort.

En effet, une incompatibilité de ces huiles avec le polymère dans la composition peut diminuer son efficacité vis-à-vis de la tenue et/ou le rendre collant. Elles peuvent aussi le solubiliser de manière trop importante, ce qui l'empêche de former un film après application et diminue là encore son influence sur la tenue.

Il reste donc nécessaire de trouver des associations optimales entre des polymères et des huiles non volatiles.

Les inventeurs ont trouvé de manière inattendue que l'association d'un copolymère séquencé tel que défini ci-après, avec une huile non volatile spécifique et une huile volatile, permet d'obtenir des propriétés de tenue optimisées, et avantageusement des propriétés de confort.

Comme le polymère, l'huile volatile permet d'améliorer les propriétés de tenue de la composition. Elle peut être hydrocarbonée, siliconée ou encore fluorée, de préférence hydrocarbonée. Elle peut aussi constituer un milieu permettant de véhiculer le polymère avant de l'introduire dans la composition.

L'huile non volatile spécifique de l'invention est hydrocarbonée, elle est caractérisée par un paramètre de solubilité de Hansen $\delta a$ compris entre 2 et 7 $(J/cm^3)^{1/2}$ et une masse moléculaire inferieure ou égale à 300 g/mol. Sans être tenue par une quelconque théorie, lorsque l'huile non volatile possède un paramètre de solubilité $\delta a$ en dehors de cette fourchette, elle n'est pas assez compatible avec le polymère et défavorise les propriétés de tenue apportée par le polymère.

De manière préférentielle le ratio du polymère sur l'huile non volatile est compris entre 0,5 et 100 et de manière encore plus préférentielle entre 1 et 40.

On connaît de l'art antérieur l'utilisation de copolymères tels que décrits ci-après dans la présente invention, dans des compositions cosmétiques de soin ou de maquillage, notamment des rouges à lèvres ayant de bonnes propriétés de tenue (EP1 411 069 de L'Oreal ou EP1 518 535 de L'Oreal).

Mais à la connaissance de la Demanderesse, il n'a jamais été décrit de les associer spécifiquement à des huiles non volatiles hydrocarbonées caractérisées par un paramètre de solubilité de Hansen $\delta a$ allant de 2 à 7 $(J/cm^3)^{1/2}$ et une masse moléculaire inférieure ou égale à 300 g/mol, pour obtenir des produits ayant des propriétés améliorées en terme de tenue et de confort après application.

L'invention concerne donc une composition cosmétique de soin ou de maquillage des matières kératiniques comprenant au moins une phase grasse liquide et au moins :

a) un copolymère éthylénique séquencé (également appelé polymère éthylénique séquencé), contenant au moins une première séquence ayant une température de transition vitreuse (Tg) supérieure ou égale à 40°C et étant issue en totalité ou en partie de un ou plusieurs premiers monomères, qui sont tels que l'homopolymère préparé à partir de ces monomères a une température de transition vitreuse supérieure ou égale à 40°C, et au moins une deuxième séquence ayant une température de transition vitreuse inférieure ou égale à 20°C et étant issue en totalité ou en partie de un ou plusieurs deuxièmes monomères, qui sont tels que l'homopolymère préparé à partir de ces monomères a une température de transition vitreuse inférieure ou égale à 20°C, ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité 1 supérieur à 2,
b) une huile non volatile hydrocarbonée caractérisée par un paramètre de solubilité de Hansen $\delta a$ allant de 2 à 7 (J/cm3)1/2 et une masse moléculaire inférieure ou égale à 300g/mol, et
c) une huile volatile.

De préférence, le copolymère séquencé (a) et l'huile non volatile spécifique (b) sont présents dans ladite

composition en des teneurs telles que le rapport pondéral (a) / (b) va de 0,5 à 100, de préférence de 1 à 40.

De préférence, ladite composition est sous la forme d'une émulsion, de préférence d'une émulsion eau-dans-huile (E/H).

Selon un mode particulier, il s'agit d'un fond de teint.

L'association selon l'invention permet en outre de formuler des compositions sous la forme de dispersions ou d'émulsions sans composés siliconés.

L'invention concerne également un procédé cosmétique de soin ou de maquillage des matières kératiniques comprenant l'application sur lesdites matières kératiniques, et notamment la peau d'une composition selon l'invention.

## COPOLYMERES SEQUENCES

La composition de l'invention comprend au moins un copolymère séquencé.

Ces polymères comprennent au moins une première séquence et au moins une deuxième séquence, incompatibles entre elles, et reliées par une séquence intermédiaire qui comprend au moins un monomère constitutif de chacune des dites deux séquences. Ces monomères sont de préférence de type acrylates ou méthacrylates d'alkyles, notamment de méthyle, d'isobutyle, d'isobornyle ou d'acide (méth) acrylique.

Ces polymères sont généralement véhiculables dans des solvants volatils ou non volatils en particulier les alcanes tels que l'isododécane.

Dans cette famille, on peut citer, de manière préférentielle, le copolymère poly (acrylate d'isobornyle/methacrylate d'isobornyle/acrylate d'isobutyle/acide acrylique) dont la synthèse est décrite dans l'exemple 1 décrit ci-après.

Selon l'invention, le copolymère séquencé peut être présent en une teneur en matière active allant de 0,1 à 30%, de préférence entre 0,5 à 20 % et encore plus préférentiellement entre 1 à 10 % en poids de matière active de copolymère séquencé par rapport au poids total de ladite composition.

Les polymères séquencés (copolymères) utilisables selon l'invention sont par exemple ceux décrits dans la demande de brevet EP 1 411 069 A2 de l'Oréal.

Ces copolymères comprennent une première et au moins une deuxième séquence, lesdites première et deuxième séquence étant reliées par une séquence intermédiaire qui comprend au moins un monomère constitutif de chacune desdites deux séquences.

La composition selon l'invention comprend donc au moins un copolymère éthylénique séquencé (également appelé polymère éthylénique séquencé), contenant au moins une première séquence ayant une température de transition vitreuse (Tg) supérieure ou égale à 40°C et étant issue en totalité ou en partie de un ou plusieurs premiers monomères, qui sont tels que l'homopolymère préparé à partir de ces monomères a une température de transition vitreuse supérieure ou égale à 40°C, et au moins une deuxième séquence ayant une température de transition vitreuse inférieure ou égale à 20°C et étant issue en totalité ou en partie de un ou plusieurs deuxièmes monomères, qui sont tels que l'homopolymère préparé à partir de ces monomères a une température de transition vitreuse inférieure ou égale à 20°C, ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité I supérieur à 2.

Le polymère séquencé utilisé selon l'invention comprend ainsi au moins une première séquence et au moins une deuxième séquence.

Par "au moins" une séquence, on entend une ou plusieurs séquences.

Par polymère "séquencé", on entend un polymère comprenant au moins 2 séquences distinctes, de préférence au moins 3 séquences distinctes.

Par polymère "éthylénique", on entend un polymère obtenu par polymérisation de monomères comprenant une insaturation éthylénique.

Le polymère éthylénique séquencé utilisé selon l'invention est préparé exclusivement à partir de monomères monofonctionnels.

Cela signifie que le polymère éthylénique séquencé utilisé selon la présente invention ne contient pas de monomères multifonctionnels, qui permettent de casser la linéarité d'un polymère afin d'obtenir un polymère branché ou voire réticulé, en fonction du taux de monomère multifonctionnel. Le polymère utilisé selon l'invention ne contient pas non plus de macromonomères (par « macromonomère » on entend un monomère monofonctionnel ayant un groupe pendant de nature polymérique, et ayant de préférence une masse moléculaire supérieure à 500 g/mol, ou bien un polymère comportant sur une seule de ses extrémités un groupe terminal polymérisable (ou à insaturation éthylénique)), qui sont utilisés à la préparation d'un polymère greffé.

On précise que dans ce qui précède et ce qui suit les termes "première" et "deuxième" séquences ne conditionnent nullement l'ordre desdites séquences (ou blocs) dans la structure du polymère.

La première séquence et la deuxième séquence du polymère utilisé dans l'invention peuvent être avantageusement incompatibles l'une avec l'autre.

Par "séquences incompatibles l'une avec l'autre", on entend que le mélange formé par un polymère correspondant à la première séquence et par un polymère correspondant à la deuxième séquence, n'est pas miscible dans le solvant de polymérisation, majoritaire en poids, du polymère séquencé, à température ambiante (25°C) et pression atmosphérique ($10^5$ Pa), pour une teneur du mélange desdits polymères supérieure ou égale à 5 % en poids, par rapport au poids total du mélange desdits polymères et dudit solvant de polymérisation, étant entendu que :

i) lesdits polymères sont présents dans le mélange en une teneur telle que le rapport pondéral respectif va de 10/90 à 90/10, et que

ii) chacun des polymères correspondant au première et seconde séquences a une masse moléculaire moyenne (en poids ou en nombre) égale à celle du polymère séquencé +/- 15%.

Dans le cas d'un mélange de solvants de polymérisation, dans l'hypothèse de deux ou plusieurs solvants présents en proportions massiques identiques, ledit mélange de polymères est non miscible dans au moins l'un d'entre eux.

Bien entendu, dans le cas d'une polymérisation réalisée dans un solvant unique, ce dernier est le solvant majoritaire.

Le polymère séquencé selon l'invention comprend au moins une première séquence et au moins une deuxième séquence reliées entre elles par un segment intermédiaire comprenant au moins un monomère constitutif de la première séquence et au moins un monomère constitutif de la deuxième séquence. Le segment intermédiaire (également appelé séquence intermédiaire) a une température de transition vitreuse Tg comprise entre les températures de transition vitreuse des première et deuxième séquences.

Le segment intermédiaire est une séquence comprenant au moins un monomère constitutif de la première séquence et au moins un monomère constitutif de la deuxième séquence du polymère permet de "compatibiliser" ces séquences.

Le segment intermédiaire comprenant au moins un monomère constitutif de la première séquence et au moins un monomère constitutif de la deuxième séquence du polymère est un polymère statistique.

De préférence, la séquence intermédiaire est issue essentiellement de monomères constitutifs de la première séquence et de la deuxième séquence.

Par "essentiellement", on entend au moins à 85%, de préférence au moins à 90%, mieux à 95% et encore mieux à 100%.

Le polymère séquencé selon l'invention est avantageusement un polymère éthylénique séquencé filmogène.

Par polymère "éthylénique", on entend un polymère obtenu par polymérisation de monomères comprenant une insaturation éthylénique.

Par polymère "filmogène", on entend un polymère apte à former à lui seul ou en présence d'un agent auxiliaire de filmification, un dépôt continu sur un support, notamment sur les matières kératiniques.

De façon préférentielle, le polymère selon l'invention ne comprend pas d'atomes de silicium dans son squelette. Par "squelette", on entend la chaîne principale du polymère, par opposition aux chaînes latérales pendantes.

De préférence, le polymère selon l'invention n'est pas hydrosoluble, c'est à dire que le polymère n'est pas soluble dans l'eau ou dans un mélange d'eau et de monoalcools inférieurs linéaires ou ramifiés ayant de 2 à 5 atomes de carbone comme l'éthanol, l'isopropanol ou le n-propanol, sans modification de pH, à une teneur en matière active d'au moins 1% en poids, à température ambiante (25°C).

De préférence, le polymère selon l'invention n'est pas un élastomère.

Par "polymère non élastomère", on entend un polymère qui, lorsqu'il est soumis à une contrainte visant à l'étirer (par exemple de 30% relativement à sa longueur initiale), ne revient pas à une longueur sensiblement identique à sa longueur initiale lorsque cesse la contrainte.

De manière plus spécifique, par "polymère non élastomére" on désigne un polymère ayant une recouvrance instantanée $R_i$ < à 50% et une recouvrance retardée $R_{2h}$ < 70% après avoir subi un allongement de 30%. De préférence, $R_i$ est < à 30 %, et $R_{2h}$ < 50%.

Plus précisément, le caractère non élastomérique du polymère est déterminé selon le protocole suivant :

On prépare un film de polymère par coulage d'une solution du polymère dans une matrice téflonnée puis séchage pendant 7 jours dans une ambiance contrôlée à 23±5°C et 50±10 % d'humidité relative.

On obtient alors un film d'environ 100 µm d'épaisseur dans lequel sont découpées des éprouvettes rectangulaires (par exemple à l'emporte-pièce) d'une largeur de 15 mm et d'une longueur de 80 mm.

On impose à cet échantillon une sollicitation de traction à l'aide d'un appareil commercialisé sous la référence Zwick, dans les mêmes conditions de température et d'humidité que pour le séchage.

Les éprouvettes sont étirées à une vitesse de 50 mm/min et la distance entre les mors est de 50 mm, ce qui correspond à la longueur initiale ($l_0$) de l'éprouvette.
On détermine la recouvrance instantanée Ri de la manière suivante :

- on étire l'éprouvette de 30 % ($\varepsilon_{max}$) c'est-à-dire environ 0,3 fois sa longueur initiale ($l_0$)
- on relâche la contrainte en imposant une vitesse de retour égale à la vitesse de traction, soit 50 mm/min et on mesure l'allongement résiduel de l'éprouvette en pourcentage, après retour à contrainte charge nulle ($\varepsilon_i$).

La recouvrance instantanée en % ($R_i$) est donnée par la formule ci-après:

$$R_i = (\varepsilon_{max} - \varepsilon_i)/ \varepsilon_{max}) \times 100$$

Pour déterminer la recouvrance retardée, on mesure après 2 heures le taux d'allongement résiduel de l'éprouvette en pourcentage ( $_{2h}$), 2 heures après retour à la contrainte charge nulle.
La recouvrance retardée en % ($R_{2h}$) est donnée par la formule ci-après:

$$R_{2h}= (\varepsilon_{max} - \varepsilon_{2h})/ \varepsilon_{max}) \times 100$$

A titre purement indicatif, un polymère selon un mode de réalisation de l'invention possède de préférence une recouvrance instantanée $R_i$ de 10% et une recouvrance retardée $R_{2h}$ de 30%.
L'indice de polydispersité du polymère de l'invention est supérieur à 2.
Avantageusement, le polymère séquencé utilisé dans les compositions selon l'invention a un indice de polydispersité 1 supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.
L'indice de polydispersité 1 du polymère est égal au rapport de la masse moyenne en poids Mw sur la masse moyenne en nombre Mn.
On détermine les masses molaires moyennes en poids (Mw) et en nombre (Mn) par chromatographie liquide par perméation de gel (solvant THF, courbe d'étalonnage établie avec des étalons de polystyrène linéaire, détecteur réfractométrique).
La masse moyenne en poids (Mw) du polymère selon l'invention est de préférence inférieure ou égale à 300 000, elle va par exemple de 35 000 à 200 000, et mieux de 45 000 à 150 000 g/mol.
La masse moyenne en nombre (Mn) du polymère selon l'invention est de préférence inférieure ou égale à 70 000, elle va par exemple de 10 000 à 60 000, et mieux de 12 000 à 50 000 g/mol.
De préférence, l'indice de polydispersité du polymère selon l'invention est supérieur à 2, par exemple allant de 2 à 9, de préférence supérieur ou égal à 2,5, par exemple allant de 2,5 à 8, et mieux supérieur ou égal à 2,8 et notamment, allant de 2,8 à 6.

## Première séquence avant une Tg supérieure ou égale à 40°C

La séquence ayant une Tg supérieure ou égale à 40°C a par exemple une Tg allant de 40 à 150°C, de préférence supérieure ou égale à 50°C, allant par exemple de 50°C à 120 °C, et mieux supérieure ou égale à 60°C, allant par exemple de 60°C à 120°C.
Les températures de transition vitreuse indiquées des première et deuxième séquences peuvent être des Tg théoriques déterminées à partir des Tg théoriques des monomères constitutifs de chacune des séquences, que l'on peut trouver dans un manuel de référence tel que le Polymer Handbook, 3rd ed, 1989, John Wiley, selon la relation suivante, dite Loi de Fox :

$$1/Tg = \sum_i (\varpi_i / Tg_i) \, ,$$

$\varpi_i$, étant la fraction massique du monomère i dans la séquence considerée et $Tg_i$ étant la température de transition vitreuse de l'homopolymère du monomère i.

Sauf indication contraire, les Tg indiquées pour les première et deuxième séquences dans la présente demande sont des Tg théoriques.

L'écart entre les températures de transition vitreuse des première et deuxième séquences est généralement supérieur à 10°C, de préférence supérieur à 20°C, et mieux supérieur à 30°C.

On entend désigner dans la présente invention, par l'expression « compris entre ... et ... », un intervalle de valeurs dont les bornes mentionnées sont exclues, et « de ... à ... » et « allant de ... à ... », un intervalle de valeurs dont les bornes sont inclues.

La séquence ayant une Tg supérieure ou égale à 40°C peut être un homopolymère ou un copolymère.

La séquence ayant une Tg supérieure ou égale à 40°C peut être issue en totalité ou en partie de un ou plusieurs monomères, qui sont tels que l'homopolymère préparé à partir de ces monomères a une température de transition vitreuse supérieure ou égale à 40°C. Cette séquence peut également être appelée « séquence rigide ».

Dans le cas où cette séquence est un homopolymère, elle est issue de monomères, qui sont tel(s) que les homopolymères préparés à partir de ces monomères ont des températures de transition vitreuse supérieures ou égales à 40°C. Cette première séquence peut être un homopolymère, constitué par un seul type de monomère (dont la Tg de l'homopolymère correspondant est supérieure ou égale à 40°C).

Dans le cas où la première séquence est un copolymère, elle peut être issue en totalité ou en partie de un ou de plusieurs monomères, dont la nature et la concentration sont choisies de façon que la Tg du copolymère résultant soit supérieure ou égale à 40°C. Le copolymère peut par exemple comprendre :

- des monomères qui sont tel(s) que les homopolymères préparés à partir de ces monomères ont des Tg supérieures ou égales à 40°C, par exemple une Tg allant de 40°C à 150 °C, de préférence supérieure ou égale à 50°C, allant par exemple de 50°C à 120°C, et mieux supérieure ou égale à 60°C, allant par exemple de 60°C à 120°C, et
- des monomères qui sont tel(s) que les homopolymères préparés à partir de ces monomères ont des Tg inférieures à 40°C, choisis parmi les monomères ayant une Tg comprise entre 20°C à 40°C et/ou les monomères ayant une Tg inférieure ou égale à 20°C, par exemple une Tg allant de -100°C à 20°C, de préférence inférieure à 15°C, notamment allant de - 80°C à 15°C et mieux inférieur à 10°C, par exemple allant de - 50°C à 0°C à, tels que décrits plus loin.

Les premiers monomères dont les homopolymères ont une température de transition vitreuse supérieure ou égale à 40°C sont, de préférence, choisis parmi les monomères suivants, appelés aussi monomères principaux :

- les méthacrylates de formule $CH_2 = C(CH_3)\text{-}COOR_1$
  dans laquelle $R_1$ représente un groupe alkyle non substitué, linéaire ou ramifié, contenant de 1 à 4 atomes de carbone, tel qu'un groupe méthyle, éthyle, propyle ou isobutyle ou $R_1$ représente un groupe cycloalkyle $C_4$ à $C_{12}$, de préférence un cycloalkyle $C_8$ à $C_{12}$, tel que le méthacrylate d'isobornyle,

- les acrylates de formule $CH_2 = CH\text{-}COOR_2$
  dans laquelle $R_2$ représente un groupe cycloalkyle en $C_4$ à $C_{12}$ tel qu'un groupe isobornyle ou un groupe tertio butyle,

- les (méth)acrylamides de formule :

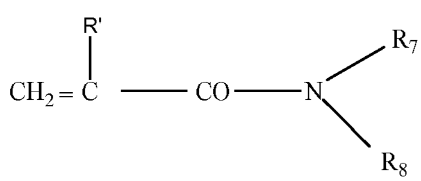

où $R_7$ et $R_8$ identiques ou différents représentent chacun un atome d'hydrogène ou un groupe alkyle en $C_1$ à $C_{12}$ linéaire ou ramifié, tel qu'un groupe n-butyle, t-butyle, isopropyle, isohexyle, isooctyle, ou isononyle ; ou $R_7$ représente H et $R_8$ représente un groupement 1,1-diméthyl-3-oxobutyl,
et R' désigne H ou méthyle. Comme exemple de monomères, on peut citer le N-butylacrylamide, le N-t-butylacrylamide, le N-isopropylacrylamide, le N,N-diméthylacrylamide et le N,N-dibutylacrylamide ,

- et leurs mélanges.

La première séquence est avantageusement obtenue à partir d'au moins un monomère acrylate de formule $CH_2$ = CH-$COOR_2$ et d'au moins un monomère méthacrylate de formule $CH_2$ = $C(CH_3)$-$COOR_2$ dans laquelle $R_2$ représente un groupe cycloalkyle $C_4$ à $C_{12}$, de préférence un cycloalkyle $C_8$ à $C_{12}$, tel que l'isobornyle. Les monomères et leurs proportions sont de préférence choisis de telle sorte que la température de transition vitreuse de la première séquence est supérieure ou égale à 40°C.

Selon un mode de mise en oeuvre, la première séquence est obtenue à partir :

i) d'au moins un monomère acrylate de formule $CH_2$ = CH-$COOR_2$ dans laquelle $R_2$ représente un groupe cycloalkyle $C_4$ à $C_{12}$, de préférence un groupe cycloalkyle en $C_8$ à $C_{12}$, tel que l'isobornyle,
ii) et d'au moins un monomère méthacrylate de formule $CH_2$ = $C(CH_3)$-COOR'$_2$ dans laquelle R'$_2$ représente un groupe cycloalkyle $C_4$ à $C_{12}$, de préférence un groupe cycloalkyle en $C_8$ à $C_{12}$, tel que l'isobornyle.

Selon un mode de mise en oeuvre, la première séquence est obtenue à partir d'au moins un monomère acrylate de formule $CH_2$ = CH-$COOR_2$ dans laquelle $R_2$ représente un groupe cycloalkyle $C_8$ à $C_{12}$, tel que l'isobornyle, et d'au moins un monomère méthacrylate de formule $CH_2$ = $C(CH_3)$-COOR'$_2$ dans laquelle R'$_2$ représente un groupe cycloalkyle $C_8$ à $C_{12,}$, tel que l'isobornyle.

De façon préférée, $R_2$ et R'$_2$ représentent indépendamment ou simultanément un groupe isobornyle.
De façon préférée, le copolymère séquencé comprend de 50 à 80 % en poids de méthacrylate/acrylate d'isobornyle, de 10 à 30 % en poids d'acrylate d'isobutyle et de 2 à 10 % en poids d'acide acrylique.

La première séquence peut être obtenue exclusivement à partir dudit monomère acrylate et dudit monomère méthacrylate.

Le monomère acrylate et le monomère méthacrylate sont de préférence dans des propositions massiques comprises entre 30 :70 et 70 :30, de préférence entre 40 :60 et 60 :40, notamment de l'ordre de 50 :50.

La proportion de la première séquence va avantageusement de 20 à 90% en poids du polymère, mieux de 30 à 80% et encore mieux de 60 à 80%.

Selon un mode de mise en oeuvre, la première séquence est obtenue par polymérisation du méthacrylate d'isobornyle et de l'acrylate d'isobornyle.

**Deuxième séquence de température de transition vitreuse inférieure à 20°C.**

La deuxième séquence a avantageusement une température de transition vitreuse Tg inférieure ou égale à 20°C a par exemple une Tg allant de -100°C à 20°C, de préférence inférieure ou égale à 15°C, notamment allant de -80°C à 15°C et mieux inférieure ou égale à 10°C, par exemple allant de - 100 °C à 10 °C, notamment allant de -30°C à 10°C.

La deuxième séquence est issue en totalité ou en partie de un ou plusieurs deuxièmes monomères, qui sont tels que l'homopolymère préparé à partir de ces monomères a une température de transition vitreuse inférieure ou égale à 20°C.

Cette séquence peut également être appelée « séquence souple ».

Le monomère ayant une Tg inférieure ou égale à 20°C (appelé deuxième monomère) est, de préférence, choisi parmi les monomères suivants:

- les acrylates de formule $CH_2$ = $CHCOOR_3$, $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule $CH_2$ = $C(CH_3)$-$COOR_4$, $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5$-CO-O-CH = $CH_2$ où $R_5$ représente un groupe alkyle en $C_4$ à $C_{12}$ linéaire ou ramifié ;
- les éthers d'alcool vinylique et d'alcool en $C_4$ à $C_{12}$,
- les N-alkyl en $C_4$ à $C_{12}$ acrylamides, tels que le N-octylacrylamide,
- et leurs mélanges.

Les monomères ayant une Tg inférieure ou égale à 20˚C préférés sont l'acrylate d'isobutyle, l'acrylate d'éthyl-2 hexyle ou leurs mélanges en toutes proportions.

Chacune des première et deuxième séquences peut contenir, en proportion minoritaire, au moins un monomère constitutif de l'autre séquence.

Ainsi la première séquence peut contenir au moins un monomère constitutif de la deuxième séquence et inversement.

Selon un mode préféré de l'invention, le ou lesdits premiers monomères, qui sont tels que l'homopolymère préparé à partir de ces monomères a une températures de transition vitreuse supérieure ou égale à 40˚C, sont choisis parmi :

- les méthacrylates de formule $CH_2 = C(CH_3)\text{-}COOR$,
  dans laquelle $R_1$ représente un groupe alkyle non substitué, linéaire ou ramifié, contenant de 1 à 4 atomes de carbone, un groupe cycloalkyle $C_4$ à $C_{12}$,,
- les acrylates de formule $CH_2 = CH\text{-}COOR_2$
  dans laquelle $R_2$ représente un groupe cycloalkyle en $C_4$ à $C_{12}$,
- les (méth)acrylamides de formule :

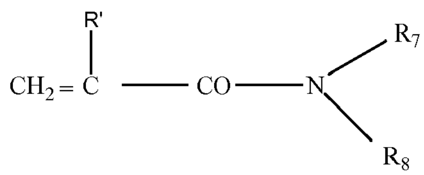

où $R_7$ et $R_8$ identiques ou différents représentent chacun un atome d'hydrogène ou un groupe alkyle en $C_1$ à $C_{12}$ linéaire ou ramifié, ou $R_7$ représente H et $R_8$ représente un groupement 1,1-diméthyl-3-oxobutyl,
et R' désigne H ou méthyle.
et le ou lesdits deuxièmes monomères, qui sont tels que l'homopolymère préparé à partir de ces monomères a une température de transition vitreuse inférieure ou égale à 20˚C, sont choisis parmi :

- les acrylates de formule $CH_2 = CHCOOR_3$,
  $R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,
- les méthacrylates de formule $CH_2 = C(CH_3)\text{-}COOR_4$,
  $R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;
- les esters de vinyle de formule $R_5\text{-}CO\text{-}O\text{-}CH = CH_2$
  où $R_5$ représente un groupe alkyle en $C_4$ à $C_{12}$ linéaire ou ramifié ;
- les éthers d'alcool vinylique et d'alcool en $C_4$ à $C_{12}$,
- les N-alkyl en $C_4$ à $C_{12}$ acrylamides, tels que le N-octylacrylamide,
- et leurs mélanges.

Chacune des première et/ou deuxième séquence, comprennent, outre les monomères indiqués ci-dessus, un ou plusieurs autres monomères appelés monomères additionnels, différents des monomères principaux cités précédemment. En particulier, chacune des première et/ou deuxième séquence comprennent au moins un monomère additionnel. La nature et la quantité de ce ou ces monomères additionnels sont choisies de manière à ce que la séquence dans laquelle ils se trouvent ait la température de transition vitreuse désirée.

Ce monomère additionnel est par exemple choisi parmi :

- les monomères à insaturation(s) éthylénique(s) comprenant au moins une fonction amine tertiaire comme la 2-vinylpyridine, la 4-vinylpyridine, le méthacrylate de diméthylaminoéthyle, le méthacrylate de diéthylaminoéthyle, le diméthylaminopropyl méthacrylamide et les sels de ceux-ci,

- les méthacrylates de formule $CH_2 = C(CH_3)\text{-}COOR_6$ dans laquelle $R_6$ représente un groupe alkyle linéaire ou ramifié, contenant de 1 à 4 atomes de carbone, tel qu'un groupe méthyle, éthyle, propyle ou isobutyle, ledit groupe alkyle étant substitué par un ou plusieurs substituants choisis parmi les groupes hydroxyle (comme le méthacrylate de 2-hydroxypropyle, le méthacrylate de 2-hydroxyéthyle) et les atomes d'halogènes (Cl, Br, I, F), tel que le méthacrylate

de trifluoroéthyle,

- les méthacrylates de formule $CH_2 = C(CH_3)\text{-}COOR_9$,
  $R_9$ représentant un groupe alkyle en $C_6$ à $C_{12}$ linéaire ou ramifié, dans lequel se trouve(nt) éventuellement intercalé(s) un ou plusieurs hétéroatomes choisis parmi 0, N et S, ledit groupe alkyle étant substitué par un ou plusieurs substituants choisis parmi les groupes hydroxyle et les atomes d'halogènes (Cl, Br, I, F) ;

- les acrylates de formule $CH_2 = CHCOOR_{10}$,
  $R_{10}$ représentant un groupe alkyle en $C_1$ à $C_{12}$ linéaire ou ramifié substitué par un ou plusieurs substituants choisis parmi les groupes hydroxyle et les atomes d'halogène (Cl, Br, I et F), tel que l'acrylate de 2-hydroxypropyle et l'acrylate de 2-hydroxyéthyle, ou $R_{10}$ représente un alkyle en $C_1$ à $C_{12}$-O-POE (polyoxyéthylène) avec répétition du motif oxyéthylène de 5 à 10 fois, par exemple méthoxy-POE, ou $R_8$ représente un groupement polyoxyéthyléné comprenant de 5 à 10 motifs d'oxyde d'éthylène.

- En particulier, la première séquence et/ou la deuxième séquence comprend à titre de monomère additionnel de l'acide (meth)acrylique, de préférence de l'acide acrylique.

Le(s) monomère(s) additionnel(s) peu(ven)t représenter de 1 à 60% en poids par rapport au poids total du polymère, de préférence de 3 à 30% en poids par rapport au poids total dudit polymère.
En particulier, ledit copolymère séquencé est tel que

la première séquence est obtenue à partir d'au moins un monomère acrylate de formule $CH_2 = CH\text{-}COOR_2$ dans laquelle $R_2$ représente un groupe cycloalkyle $C_4$ à $C_{12}$ et d'au moins un monomère méthacrylate de formule $CH_2 = C(CH_3)\text{-}COOR'_2$ dans laquelle $R'_2$ représente un groupe cycloalkyle $C_4$ à $C_{12}$, et
la deuxième séquence est obtenue à partir d'au moins un deuxième monomère tel que l'homopolymère obtenu a une température de transition vitreuse inférieure ou égale à 20 °C, et d'un monomère additionnel de type acide acrylique.

De préférence, le polymère de l'invention comprend au moins des monomères acrylate d'isobornyle et méthacrylate d'isobornyle dans la première séquence et des monomères acrylate d'isobutyle et acide acrylique dans la deuxième séquence.
De préférence, le polymère comprend au moins des monomères acrylate d'isobornyle et méthacrylate d'isobornyle en proportion équivalente en poids dans la première séquence et des monomères acrylate d'isobutyle et acide acrylique dans la deuxième séquence.
De préférence, le polymère comprend au moins des monomères acrylate d'isobornyle et méthacrylate d'isobornyle en proportion équivalente en poids dans la première séquence, et des monomères acrylate d'isobutyle et acide acrylique dans la deuxième séquence, la première séquence représentant 70% en poids du polymère.
De préférence, le polymère comprend au moins des monomères acrylate d'isobornyle et méthacrylate d'isobornyle en proportion équivalente en poids dans la première séquence, et des monomères acrylate d'isobutyle et acide acrylique dans la deuxième séquence. De façon préférée, la séquence de Tg supérieure à 40°C représentant 70% en poids du polymère, et l'acide acrylique représentant 5% en poids du polymère.
Selon un mode de réalisation, la première séquence ne comprend pas de monomère additionnel.
Selon un mode de réalisation préféré, la deuxième séquence comprend de l'acide acrylique à titre de monomère additionnel. En particulier, la deuxième séquence est avantageusement obtenue à partir d'un monomère acide acrylique et d'au moins un autre monomère ayant une Tg inférieure ou égale à 20°C.
Selon un mode de réalisation préféré, l'invention concerne une composition cosmétique pour le maquillage et/ou le soin des matières kératiniques comprenant, dans un milieu physiologiquement acceptable, au moins un copolymère comprenant au moins un monomère acrylate de formule $CH_2 = CH\text{-}COOR_2$ dans laquelle $R_2$ représente un groupe cycloalkyle $C_8$ à $C_{12}$ et/ou au moins un monomère méthacrylate de formule $CH_2 = C(CH_3)\text{-}COOR'_2$ dans laquelle $R'_2$ représente un groupe cycloalkyle $C_8$ à $C_{12}$, au moins un deuxième monomère acrylate de formule $CH_2 = CHCOOR_3$, dans laquelle $R_3$ représente un groupe alkyle non substitué en $C_1$ à $C_{12}$, linéaire ou ramifié, à l'exception du groupe tertiobutyle, et au moins un monomère acide acrylique.
De façon préférée, le copolymère utilisé dans les compositions selon l'invention est obtenu à partir d'au moins un monomère méthacrylate d'isobornyle, d'au moins un monomère acrylate d'isobornyle, d'au moins un monomère acrylate d'isobutyle et d'au moins un monomère d'acide acrylique.
Avantageusement, le copolymère utilisé dans l'invention comprend de 50 à 80 % en poids de mélange méthacrylate/acrylate d'isobornyle, de 10 à 30 % en poids d'acrylate d'isobutyle et de 2 à 10 % en poids d'acide acrylique.
Le copolymère séquencé peut avantageusement comprendre plus de 2 % en poids de monomères acide

acrylique, et notamment de 2 à 15 % en poids, par exemple de 3 à 15 % en poids, en particulier de 4 à 15 % en poids, voire de 4 à 10 % en poids de monomères acide acrylique, par rapport au poids total dudit copolymère.

**Segment intermédiaire**

Le segment intermédiaire (également appelé séquence intermédiaire) relie la première séquence et la deuxième séquence du polymère utilisé selon la présente invention. Le segment intermédiaire résulte de la polymérisation :

i) du ou des premiers monomères, et éventuellement du ou des monomères additionnels, restant disponibles après leur polymérisation à un taux de conversion d'au maximum 90% pour former la première séquence,
ii) et du ou des deuxièmes monomères, et éventuellement du ou des monomères additionnels, ajoutés dans le mélange réactionnel.

La formation de la deuxième séquence est initiée lorsque les premiers monomères ne réagissent plus ou ne s'incorporent plus dans la chaine polymérique soit parce qu'ils sont tous consommés soit parce que leur réactivité ne leur permet plus d'être.
Ainsi le segment intermédiaire comprend les premiers monomères disponibles, résultant d'un taux de conversion de ces premiers monomères inférieur ou égal à 90%, lors de l'introduction du ou des deuxièmes monomères lors de la synthèse du polymère.
Le segment intermédiaire du polymère séquencé est un polymère statistique (peut également être appelé une séquence statistique). C'est-à-dire qu'il comprend une répartition statistique du ou des premiers monomères et du ou des deuxièmes monomères ainsi que du ou des monomères additionnels éventuellement présents.
Ainsi, le segment intermédiaire est une séquence statistique, de même que la première séquence et la deuxième séquence si elles ne sont pas des homopolymères (c'est-à-dire si elles sont toutes deux formées à partir d'au moins deux monomères différents).

**Procédé de préparation du copolymère :**

Le copolymère éthylénique séquencé selon l'invention est préparé par polymérisation radicalaire libre, selon les techniques bien connues de ce type de polymérisation.
La polymérisation radicalaire libre est effectuée en présence d'un amorceur dont la nature est adaptée, de façon connue, en fonction de la température de polymérisation souhaitée et du solvant de polymérisation. En particulier, l'amorceur peut être choisi parmi les amorceurs à fonction peroxyde, les couples d'oxydoréduction, ou d'autres amorceurs de polymérisation radicalaire connus de l'homme de l'art.
En particulier, à titre d'amorceur à fonction peroxyde, on peut citer par exemple:

a. les péroxyesters, tel que le terbutyl-péroxyacétate, le perbenzoate de tertiobutyle, le tertbutyl péroxy-2-éthylhexanoate (Trigonox 21 S d'Akzo Nobel), le 2,5-bis(2-éthylhexanoylpéroxy)-2,5-diméthylhexane (Trigonox 141 d'Akzo Nobel) ;
b. les péroxydicarbonates, tel que le di-isopropylpéroxydicarbonate ;
c. les péroxycetones, tel que le méthyléthylcétone péroxyde ;
d. hydropéroxydes, tel que l'eau oxygénée ($H_2O_2$), le terbutylhydropéroxyde ;
e. les peroxydes de diacyle, tel que l'acétyl péroxyde, le benzoyl péroxyde ;
f. les péroxydes de dialkyle, tel que le di-tertiobutyle péroxyde ;
g. les péroxydes inorganiques, tel que le péroxodisulfate de potassium ($K_2S_2O_8$);

A titre d'amorceur sous forme de couple d'oxydoréduction, on peut citer le couple thiosulfate de potassium + peroxodisulfate de potassium par exemple.
Selon un mode de réalisation préférée, l'amorceur est choisi parmi les peroxydes organiques comprenant de 8 à 30 atomes de carbone. De façon préférée, l'amorceur utilisé est le 2.5- Bis(2-éthylhexanoylperoxy)-2.5-diméthylhexane commercialisé sous la référence Trigonox® 141 par la société Akzo Nobel.
Le copolymère séquencé utilisé selon l'invention est préparé par polymérisation radicalaire libre et non par polymérisation contrôlée ou vivante. En particulier, la polymérisation du copolymère éthylénique séquencé est réalisée en l'absence d'agents de contrôle, et en particulier en l'absence d'agent de contrôle classiquement utilisés dans les procédés de polymérisation vivante ou contrôlée tels que les nitroxydes, les alcoxyamines, les dithioesters, les dithiocarbamates, les dithiocarbonates ou xanthates, les trithiocarbonates, les catalyseurs à base de cuivre, par exemple.
Comme indiqué précédemment, le segment intermédiaire est une séquence statistique, de même que la première séquence et la deuxième séquence si elles ne sont pas des homopolymères (c'est-à-dire si elles sont toutes

deux formées à partir d'au moins deux monomères différents).

Le copolymère séquencé peut être préparé par polymérisation radicalaire libre, et en particulier par un procédé consistant à mélanger, dans un même réacteur, un solvant de polymérisation, un amorceur, au moins un monomère de transition vitreuse supérieure ou égale à 40˚C, au moins un monomère de transition vitreuse inférieure ou égale à 20 ˚C selon la séquence suivante :

- on verse dans le réacteur, une partie du solvant de polymérisation et éventuellement une partie de l'amorceur et des monomères de la première coulée, mélange que l'on chauffe à une température de réaction comprise entre 60 et 120 ˚C,
- on verse ensuite, en une première coulée, ledit au moins un premier monomère de Tg supérieure ou égale à 40˚C et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, ledit au moins un deuxième monomère de transition vitreuse inférieure ou égale à 20 ˚C, qu'on laisse réagir pendant une durée T' au bout de laquelle le taux de conversion desdits monomères atteint un plateau,
- on ramène le mélange réactionnel à température ambiante.

De façon préférée, le copolymère peut être préparé par polymérisation radicalaire libre, en particulier par un procédé consistant à mélanger, dans un même réacteur, un solvant de polymérisation, un amorceur, un monomère acide acrylique, au moins un monomère de transition vitreuse inférieure ou égale à 20 ˚C, au moins un monomère acrylate de formule $CH_2$ = CH-$COOR_2$ dans laquelle $R_2$ représente un groupe cycloalkyle $C_4$ à $C_{12}$, et au moins un monomère méthacrylate de formule $CH_2$ = C($CH_3$)-COOR'$_2$ dans laquelle R'$_2$ représente un groupe cycloalkyle $C_4$ à $C_{12}$, selon la séquence d'étape suivante :

- on verse dans le réacteur, une partie du solvant de polymérisation et éventuellement une partie de l'amorceur et des monomères de la première coulée, mélange que l'on chauffe à une température de réaction comprise entre 60 et 120 ˚C,
- on verse ensuite, en une première coulée, ledit au moins monomère acrylate de formule $CH_2$ = CH-$COOR_2$ et ledit au moins monomère méthacrylate de formule $CH_2$ = C($CH_3$)-COOR'$_2$ en tant que monomères de Tg supérieure ou égale à 40˚C, et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, le monomère acide acrylique et ledit au moins monomère de transition vitreuse inférieure ou égale à 20 ˚C, qu'on laisse réagir pendant une durée T' au bout de laquelle le taux de conversion desdits monomères atteint un plateau,
- on ramène le mélange réactionnel à température ambiante.

Par solvant de polymérisation, on entend un solvant ou un mélange de solvants. En particulier, à titre de solvant de polymérisation utilisable on peut citer :

- les cétones liquides à température ambiante tels que méthyléthylcétone, méthylisobutylcétone, diisobutylcétone, l'isophorone, la cyclohexanone, l'acétone ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les éthers liquides à température ambiante tels que le diéthyléther, le diméthyléther ou le dichlorodiéthyléther ;
- les alcanes liquides à température ambiante tels que le décane, l'heptane, le dodécane, l'isododécane, le cyclohexane, l'isohexadécane ;
- les composés cycliques aromatiques liquides à température ambiante tels que le toluène et le xylène ; les aldéhydes liquides à température ambiante tels que le benzaldéhyde, l'acétaldéhyde et leurs mélanges.

Le solvant de polymérisation peut être choisi notamment parmi l'acétate d'éthyle, l'acétate de butyle, les alcools tels que l'isopropanol, l'éthanol, les alcanes aliphatiques tels que l'isododécane et leurs mélanges. De préférence, le solvant de polymérisation est un mélange acétate de butyle et isopropanol ou l'isododécane.

Selon un autre mode de mise en oeuvre, le copolymère peut être préparé par polymérisation radicalaire libre selon un procédé de préparation, consistant à mélanger, dans un même réacteur, un solvant de polymérisation, un amorceur, au moins un monomère de transition vitreuse inférieure ou égale à 20 ˚C, et au moins un monomère de Tg supérieure ou égale à 40˚C, selon la séquence d'étape suivante :

- on verse dans le réacteur, une partie du solvant de polymérisation et éventuellement une partie de l'amorceur et des monomères de la première coulée, mélange que l'on chauffe à une température de réaction comprise entre 60 et 120 °C,
- on verse ensuite, en une première coulée, ledit au moins un monomère de transition vitreuse inférieure ou égale à 20 °C et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, ledit au moins un monomère de Tg supérieure ou égale à 40°C, qu'on laisse réagir pendant une durée T' au bout de laquelle le taux de conversion desdits monomères atteint un plateau,
- on ramène le mélange réactionnel à température ambiante.

Selon un mode préféré de mise en oeuvre, le copolymère peut être préparé par polymérisation radicalaire libre selon un procédé de préparation, consistant à mélanger, dans un même réacteur, un solvant de polymérisation, un amorceur, un monomère acide acrylique, au moins un monomère de transition vitreuse inférieure ou égale à 20 °C, au moins un monomère de Tg supérieure ou égale à 40°C, et en particulier en tant que monomères de Tg supérieure ou égale à 40°C, au moins un monomère acrylate de formule $CH_2 = CH\text{-}COOR_2$ dans laquelle $R_2$ représente un groupe cycloalkyle $C_4$ à $C_{12}$, et au moins un monomère méthacrylate de formule $CH_2 = C(CH_3)\text{-}COOR'_2$ dans laquelle $R'_2$ représente un groupe cycloalkyle $C_4$ à $C_{12}$, selon la séquence d'étape suivante :

- on verse dans le réacteur, une partie du solvant de polymérisation et éventuellement une partie de l'amorceur et des monomères de la première coulée, mélange que l'on chauffe à une température de réaction comprise entre 60 et 120 °C,
- on verse ensuite, en une première coulée, le monomère acide acrylique et ledit au moins monomère de transition vitreuse inférieure ou égale à 20 °C et éventuellement une partie de l'amorceur que l'on laisse à réagir pendant une durée T correspondant à un taux de conversion desdits monomères de 90 % maximum,
- on verse ensuite dans le réacteur, en une deuxième coulée, à nouveau de l'amorceur de polymérisation, ledit au moins un monomère acrylate de formule $CH_2 = CH\text{-}COOR_2$ et ledit au moins un monomère méthacrylate de formule $CH_2 = C(CH_3)\text{-}COOR'_2$, en tant que monomère de Tg supérieure ou égale à 40°C, qu'on laisse réagir pendant une durée T' au bout de laquelle le taux de conversion desdits monomères atteint un plateau,
- on ramène le mélange réactionnel à température ambiante.

La température de polymérisation est de préférence de l'ordre de 90 °C.
La durée de réaction après la deuxième coulée est de préférence comprise entre 3 et 6 heures.
Selon un mode particulier de l'invention, on utilisera un copolymère poly (acrylate d'isobornyle/methacrylate d'isobornyle/acrylate d'isobutyle/acide acrylique) tel que préparé selon l'exemple 1 décrit ci-après.
Selon l'invention, le copolymère séquencé peut être présent en une teneur en matière active allant de 0,1 à 30%, de préférence entre 0,5 à 20 % et encore plus préférentiellement entre 1 à 10 % en poids de matière active de copolymère séquencé par rapport au poids total de ladite composition.

**PHASE GRASSE**

Une composition cosmétique conforme à la présente invention comprend au moins une phase grasse liquide comprenant au moins une huile non volatile spécifique telle que définie ci-après, et une huile volatile.
On entend par huile, tout corps gras sous forme liquide à température ambiante (20-25 °C) et à pression atmosphérique.
L'huile non volatile spécifique selon l'invention est une huile hydrocarbonée et l'huile volatile selon l'invention peut être choisie parmi les huiles hydrocarbonées, siliconées, fluorées, ou leurs mélanges.
Elles peuvent être d'origine animale, végétale, minérale ou synthétique.
Au sens de la présente invention, on entend par « huile volatile », une huile (ou milieu non aqueux) susceptible de s'évaporer au contact de la peau en moins d'une heure, à température ambiante et à pression atmosphérique. L'huile volatile est une huile cosmétique volatile, liquide à température ambiante, ayant notamment une pression de vapeur non nulle, à température ambiante et à pression atmosphérique, en particulier, ayant une pression de vapeur allant de 0,13 Pa à 40 000 Pa ($10^{-3}$ à 300 mm Hg), et de préférence, allant de 1,3 Pa à 13 000 Pa (0,01 à 100 mm Hg), et préférentiellement allant de 1,3 Pa à 1300 Pa (0,01 à 10 mm Hg).
Au sens de la présente invention, on entend par « huile non volatile », une huile ayant une pression de vapeur inférieure à 0,13 Pa.
Au sens de la présente invention, on entend par « huile siliconée », une huile comprenant au moins un atome de silicium, et notamment au moins un groupe Si-O.

On entend par « huile fluorée », une huile comprenant au moins un atome de fluor.

On entend par « huile hydrocarbonée », une huile contenant principalement des atomes d'hydrogène et de carbone.

Les huiles peuvent éventuellement comprendre des atomes d'oxygène, d'azote, de soufre et/ou de phosphore, par exemple, sous la forme de radicaux hydroxyle ou acide ou sous la forme de fonction ester.

Une composition de l'invention peut comprendre une phase grasse liquide en une teneur variant de 3 à 95 %, en particulier de 5 à 80 %, en particulier de 10 à 70 %, et plus particulièrement, de 20 à 50 % en poids par rapport au poids total de la composition.

**Huile non volatile spécifique**

La composition de l'invention comprend au moins une huile non volatile hydrocarbonée caractérisée par un paramètre de solubilité de Hansen δa allant de 2 à 7 $(J/cm^3)^{1/2}$ et une masse moléculaire inferieure ou égale à 300 g/mol.

Les huiles dont le paramètre de solubilité de Hansen δa est compris entre 4 et 6 $(J/cm^3)^{1/2}$ et dont la masse moléculaire est inférieure 250 g/mol sont préférentielles.

La définition et le calcul des paramètres de solubilité dans l'espace de solubilité tridimensionnel de HANSEN sont décrits dans l'article de C. M. HANSEN : "The three dimensionnal solubility parameters" J. Paint Technol. 39, 105 (1967).

Selon cet espace de Hansen :

- $\delta_D$ caractérise les forces de dispersion de LONDON issues de la formation de dipôles induits lors des chocs moléculaires ;
- $\delta_p$ caractérise les forces d'interactions de DEBYE entre dipôles permanents ainsi que les forces d'interactions de KEESOM entre dipôles induits et dipôles permanents;
- $\delta_h$ caractérise les forces d'interactions spécifiques (type liaisons hydrogène, acide/base, donneur/accepteur, etc.) ;
- $\delta_a$ est déterminé par l'équation : $\delta_a = (\delta_p^2 + \delta_h^2)^{½}$

Les paramètres $\delta_p$, $\delta_h$, $\delta_D$ et $\delta_a$ sont exprimés en $(J/cm^3)^{½}$.

Comme exemples d'huiles non volatiles hydrocarbonées pouvant convenir dans le cadre de cette invention, on peut citer :

L'isodecyl néopentanoate (δa: 5,32 ; M : 242,4), le myristate d'isopropyle (δa : 5,00 ; M : 270,46), le palmitate d'isopropyle (δa : 4,74 ; M : 298,51), l'ethyl-2 hexanoate d'ethyl-2 hexyle (δa : 5,15 ; M : 256,43), l'isononyl isononanoate (δa : 4,57 ; M : 284,48), l'isononanoate d'isodecyle (δa : 4,74 ; M : 298,51), le dicapryl carbonate (δa : 6,0 ; M : 286) et le dicapryl ether (δa : 3,49 ; M : 242).

Selon l'invention, l'isodecyl néopentanoate est préférentiel.

Le pourcentage d'huile non volatile est compris entre 0,1 et 30%, de préférence entre 1 et 20 % et encore plus préférentiellement entre 2 et 10 % en poids par rapport au poids total de ladite composition.

De manière préférentielle le rapport pondéral du copolymère sur l'huile non volatile est compris entre 0,5 et 100 et de manière encore plus préférentielle entre 1 et 40.

**Huile volatile**

La composition de l'invention comprend en outre au moins une huile volatile qui peut être hydrocarbonée, siliconée ou fluorée.

Les huiles volatiles peuvent être choisies parmi les huiles hydrocarbonées ayant de 8 à 16 atomes de carbone, et notamment les alcanes ramifiés en $C_8$-$C_{16}$ (appelées aussi isoparaffines), comme l'isododécane (encore appelé 2,2,4,4,6-pentaméthylheptane), l'isodécane, l'isohexadécane, et, par exemple, les huiles vendues sous les noms commerciaux d'ISOPARS® ou de PERMETHYLS®. On peut également citer les alcanes linéaires volatiles comportent de 9 à 15 atomes de carbone tels que ceux décrits dans la demande de brevet de la société Cognis WO 2007/ 068371 A1.

Comme huiles volatiles, on peut aussi utiliser les silicones volatiles, comme, par exemple, les huiles de silicones linéaires ou cycliques volatiles, notamment, celles ayant une viscosité ≤ 8 centistokes (cSt) ($8 \times 10^{-6}$ $m^2/s$), et ayant, notamment, de 2 à 10 atomes de silicium, et en particulier, de 2 à 7 atomes de silicium, ces silicones comportant, éventuellement, des groupes alkyle ou alkoxy ayant de 1 à 10 atomes de carbone. Comme huile de silicone volatile utilisable dans l'invention, on peut citer, notamment, les diméthicones de viscosité 5 et 6 cSt, l'octaméthyl cyclotétrasiloxane, le décaméthyl cyclopentasiloxane, le dodécaméthyl cyclohexasiloxane, l'heptaméthyl hexyltrisiloxane, l'octamethyl trisiloxane, le décamethyl tétrasiloxane, le dodecamethyl pentasiloxane et leurs mélanges.

On peut également utiliser des huiles volatiles fluorées, telles que le nonafluorométhoxybutane le nonafluorométhoxybutane, le décafluoropentane, le tétradécafluorohexane, le dodecafluoropentane et leurs mélanges.
De manière préférentielle, l'huile volatile est une huile volatile hydrocarbonée.
En particulier, elles peuvent être choisies parmi les huiles hydrocarbonées ayant de 8 à 14 atomes de carbone et notamment les alcanes ramifiés en $C_8$-$C_{14}$ comme les iso alcanes d'origine pétrolière comme l'isododecane et l'iso décane.
Le pourcentage d'huile volatile est compris entre 1 et 80%, de préférence entre 10 et 60 % et encore plus préférentiellement entre 20 et 50 % en poids par rapport au poids total de ladite composition.

**MILIEU PHYSIOLOGIQUEMENT ACCEPTABLE**

Par « milieu physiologiquement acceptable », on entend désigner un milieu convenant particulièrement à l'application d'une composition de l'invention sur la peau ou les lèvres.
Le milieu physiologiquement acceptable est généralement adapté à la nature du support sur lequel doit être appliquée la composition, ainsi qu'à l'aspect sous lequel la composition doit être conditionnée.
Une composition de l'invention peut être anhydre ou sous la forme d'une dispersion ou d'une émulsion.
Une émulsion peut posséder une phase continue huileuse ou aqueuse. Une telle émulsion peut être, par exemple, une émulsion inverse (E/H) ou directe (H/E), ou encore une émulsion multiple (E/H/E ou H/E/H).
Dans le cas des émulsions, les émulsions inverses (E/H) sont préférentielles.

**Agent structurant lipophile**

Une composition selon l'invention peut comprendre en outre au moins un agent structurant de phase grasse liquide choisi parmi une cire, un composé pâteux, et leurs mélanges.
En particulier, une cire convenant à l'invention peut notamment être choisie parmi des cires d'origine animale, végétale, minérale, synthétique et leurs mélanges.
A titre d'exemples de cires pouvant être utilisées selon l'invention, on peut citer

- les cires d'origine animale telles que la cire d'abeilles, le spermaceti, la cire de lanoline et les dérivés de lanoline, les cires végétales telles que la cire de Carnauba, de Candellila, d'Ouricury, du Japon, le beurre de cacao ou les cires de fibres de liège ou de canne à sucre,
- les cires minérales, par exemple de paraffine, de vaseline, de lignite ou les cires microcristallines ou les ozokérites,
- les cires synthétiques parmi lesquelles les cires de polyéthylène, et les cires obtenues par synthèse de Fisher-Tropsch,
- les cires de silicone, en particulier les polysiloxanes linéaires substitués; on peut citer, par exemple, les cires de silicone polyéther, les alkyl ou alkoxy-diméthicones ayant de 16 à 45 atomes de carbone, les alkyl méthicones comme la $C_{30}$-$C_{45}$ alkyl méthicone vendue sous la dénomination commerciale « AMS C 30 »par DOW CORNING,
- les huiles hydrogénées concrètes à 25 °C telles que l'huile de ricin hydrogénée, l'huile de jojoba hydrogénée, l'huile de palme hydrogénée, le suif hydrogéné, l'huile de coco hydrogénée et les esters gras concrets à 25 °C comme le stéarate d'alkyle en $C_{20}$-$C_{40}$ vendu sous la dénomination commerciale « KESTER WAX K82H » par la société KOSTER KEUNEN,
- et/ou leurs mélanges.

De préférence, on utilisera les cires de polyéthylène, les cires microcristallines, les cires de carnauba, l'huile de jojoba hydrogénée, les cires de candellila, les cires d'abeilles et/ou leurs mélanges.
Une composition selon l'invention peut comprendre en outre au moins un composé pâteux.
La présence d'un composé pâteux peut permettre de conférer avantageusement un confort amélioré lors du dépôt d'une composition de l'invention sur les fibres kératiniques.
Un tel composé peut être avantageusement choisi parmi la lanoline et ses dérivés ; les composés siliconés polymériques ou non ; les composés fluorés polymériques ou non ; les polymères vinyliques, notamment les homopolymères d'oléfines ; les copolymères d'oléfines ; les homopolymères et copolymères de diènes hydrogénés ; les oligomères linéaires ou ramifiés, homo ou copolymères de (méth)acrylates d'alkyles ayant de préférence un groupement alkyle en $C_8$-$C_{30}$ ; les oligomères homo et copolymères d'esters vinyliques ayant des groupements alkyle en $C_8$-$C_{30}$ ; les oligomères homo et copolymères de vinyléthers ayant des groupements alkyle en $C_8$-$C_{30}$ ; les polyéthers liposolubles résultant de la polyéthérification entre un ou plusieurs diols en $C_2$-$C_{100}$, en particulier, en $C_2$-$C_{50}$ ; les esters d'acide ou d'alcool gras ; et leurs mélanges.
Parmi les esters, on peut notamment citer :

- les esters d'un glycérol oligomère, notamment, les esters de diglycérol, comme le triisostéarate de polyglycéryl-2, les condensats d'acide adipique et de glycérol, pour lesquels une partie des groupes hydroxyle des glycérols ont réagi avec un mélange d'acides gras, tels que l'acide stéarique, l'acide caprique, l'acide stéarique et isostéarique et l'acide 12-hydroxystéarique, à l'image, notamment, de ceux commercialisé sous la marque Softisan 649 par la société Sasol ou tel que le polyacyladipate-2 de bis diglycéryle ; le propionate d'arachidyle commercialisé sous la marque Waxenol 801 par Alzo ; les esters de phytostérol ; les triglycérides d'acides gras et leurs dérivés, tels que les coco-glycérides hydrogénés ; les polyesters non réticulés résultant de la polycondensation entre un acide dicarboxylique ou un polyacide carboxylique linéaire ou ramifié en $C_4$-$C_{50}$ et un diol ou un polyol en $C_2$-$C_{50}$ ; les esters aliphatiques d'ester résultant de l'estérification d'un ester d'acide hydroxycarboxylique aliphatique par un acide carboxylique aliphatique ; les polyesters résultant de l'estérification, par un acide polycarboxylique, d'un ester d'acide hydroxy carboxylique aliphatique, ledit ester comprenant au moins deux groupes hydroxyle, tels que les produits Risocast DA-H®, et Risocast DA-L® ; et leurs mélanges.

Le ou les agents structurants peuvent être présents dans une composition de l'invention en une teneur allant de 0,1 à 30 % en poids d'agents de préférence encore de 0,5 à 20 % en poids, par rapport au poids total de la composition.

**Phase aqueuse**

La composition selon l'invention peut comprendre une phase aqueuse.
La phase aqueuse comprend de l'eau. Une eau convenant à l'invention peut être une eau florale telle que l'eau de bleuet et/ou une eau minérale telle que l'eau de VITTEL, l'eau de LUCAS ou l'eau de LA ROCHE POSAY et/ou une eau thermale.
La phase aqueuse peut également comprendre des solvants organiques miscibles à l'eau (à température ambiante -25 °C) comme par exemple les monoalcools ayant de 2 à 6 atomes de carbone tels que l'éthanol, l'isopropanol ; les polyols ayant notamment de 2 à 20 atomes de carbones, de préférence ayant de 2 à 10 atomes de carbone, et préférentiellement ayant de 2 à 6 atomes de carbone, tels que le glycérol, le propylène glycol, le butylène glycol, le pentylène glycol, l'hexylène glycol, le dipropylène glycol, le diéthylène glycol ; les éthers de glycol (ayant notamment de 3 à 16 atomes de carbone) tels que les alkyl($C_1$-$C_4$)éther de mono, di- ou tripropylène glycol, les alkyl($C_1$-$C_4$)éthers de mono, di- ou triéthylène glycol, et leurs mélanges.
La phase aqueuse peut comprendre en outre des agents de stabilisation, par exemple le chlorure de sodium, le dichlorure de magnésium et le sulfate de magnésium.
La phase aqueuse peut également comprendre tout composé hydrosoluble ou hydrodispersible compatible avec une phase aqueuse tels que des gélifiants, des polymères filmogènes, des épaississants, des tensioactifs et leurs mélanges.
Lorsque la composition de l'invention comprend une phase aqueuse, elle peut être présente en une teneur variant de 1 % à 80 % en poids, notamment de 5 % à 50 %, et plus particulièrement de 10 % à 45 % en poids par rapport au poids total de la composition.
Selon un autre mode de réalisation, une composition de l'invention peut être anhydre.
Une composition anhydre peut comprendre moins de 5 % en poids d'eau, par rapport au poids total de la composition, et en particulier moins de 3 %, notamment moins de 2 %, et plus particulièrement moins de 1 % en poids d'eau par rapport au poids total de la composition.
Plus particulièrement, une composition anhydre peut être dépourvue d'eau.

**AGENTS EPAISSISSANTS**

Selon la fluidité de la composition que l'on souhaite obtenir, on peut incorporer dans une composition de l'invention, un ou plusieurs agents épaississants ou gélifiants.
Un agent épaississant ou gélifiant convenant à l'invention peut être hydrophile, c'est-à-dire soluble ou dispersible dans l'eau.
Comme gélifiants hydrophiles, on peut citer en particulier les polymères épaississants hydrosolubles ou hydrodispersibles. Ceux-ci peuvent notamment être choisis parmi : les polymères carboxyvinyliques modifiés ou non, tels que les produits commercialisés sous les dénominations Carbopol (nom CTFA : carbomer) par la société Goodrich ; les polyacrylates et polyméthacrylates tels que les produits vendus sous les dénominations de Lubrajel et Norgel par la société GUARDIAN ou sous la dénomination Hispagel par la société HISPANO CHIMICA ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société CLARIANT sous la dénomination « Hostacerin AMPS » (nom CTFA : ammonium polyacryldimethyltauramide) ; les copolymères anioniques réticulés d'acrylamide et d'AMPS, se présentant sous la forme d'une émulsion E/H, tels ceux commercialisés sous le

nom de SEPIGEL 305 (nom C.T.F.A. : Polyacrylamide/ C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les biopolymères polysaccharidiques comme la gomme de xanthane, la gomme de guar, la gomme de caroube, la gomme d'acacia, les scléroglucanes, les dérivés de chitine et de chitosane, les carraghénanes, les gellanes, les alginates, les celluloses telles que la cellulose microcristalline, la carboxyméthylcellulose, l'hydroxyméthylcellullose et l'hydroxypropylcellulose ; et leurs mélanges.

Un agent épaississant ou gélifiant convenant à l'invention peut être lipophile. Il peut être minéral ou organique.

Comme agents épaississants lipophiles, on peut citer par exemple les argiles modifiées telles que le silicate de magnésium modifié (Bentone gel VS38 de RHEOX), les hectorites modifiées telles que l'hectorite modifiée par un chlorure d'ammonium d'acide gras en $C_{10}$ à $C_{22}$, comme l'hectorite modifiée par du chlorure de di-stéaryl di-méthyl ammonium telle que, par exemple, celle commercialisée sous la dénomination de Bentone 38V® par la société ELEMENTIS ou celle commercialisée sous la dénomination « Bentone 38 CE » par la société RHEOX ou celle commercialisée sous la dénomination Bentone Gel V5 5V par la société ELEMENTIS.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6®, KSG16® et de KSG18® par la société SHIN-ETSU, de Trefil E-505C® et Trefil E-506C® par la société DOW-CORNING, de Gransil SR-CYC®, SR DMF10®, SR-DC556®, SR 5CYC gel®, SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les polycondensats de type polyamide résultant de la condensation entre un acide dicarboxylique comprenant au moins 32 atomes de carbone et un alkylène diamine et en particulier l'éthylène diamine, dans lequel le polymère comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges. Les copolymères séquencés de type « dibloc », « tribloc » ou « radial » du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

Parmi les gélifiants lipophiles pouvant être utilisés dans une composition cosmétique de l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR, les huiles végétales hydrogénées, telles que l'huile de ricin hydrogénée, les alcools gras, en particulier de $C_8$ à $C_{26}$, et plus particulièrement de $C_{12}$ à $C_{22}$, comme par exemple, l'alcool mysritylique, l'alcool cétylique, l'alcool stéarylique ou l'alcool béhénylique.

Selon un mode de réalisation, une composition de l'invention peut comprendre des agents épaississants en une teneur en matière active de 0,01 % à 40 % en poids, notamment de 0,1 à 20 % en poids, en particulier de 1 à 15 % en poids par rapport au poids total de la composition.

## MATIERES COLORANTES

Selon un mode de réalisation préféré, la composition selon l'invention comprend outre au moins une matière colorante, en particulier au moins une matière colorante pulvérulente.

Une composition cosmétique conforme à l'invention peut, avantageusement, incorporer au moins une matière colorante choisie parmi des matières colorantes organiques ou inorganiques, notamment, de type pigments ou nacres classiquement utilisés dans les compositions cosmétiques, des colorants liposolubles ou hydrosolubles, des matériaux à effet optique spécifique et leurs mélanges.

Par pigments, il faut comprendre des particules blanches ou colorées, minérales ou organiques, insolubles dans une solution aqueuse, destinées à colorer et/ou opacifier le film résultant.

Les pigments peuvent être présents à raison de 0,1 à 40 % en poids, notamment, de 1 à 30 % en poids, et en particulier, de 5 à 15 % en poids, par rapport au poids total de la composition cosmétique.

Comme pigments minéraux utilisables dans l'invention, on peut citer les oxydes de titane, de zirconium ou de cérium, ainsi que les oxydes de zinc, de fer ou de chrome, le bleu ferrique, le violet de manganèse, le bleu outremer et l'hydrate de chrome.

Parmi les pigments organiques utilisables dans l'invention, on peut citer le noir de carbone, les pigments de

type D & C, les laques à base de carmin de cochenille, de baryum, strontium, calcium, aluminium ou encore les dicéto pyrrolopyrrole (DPP) décrits dans les documents EP-A-542 669, EP-A-787 730, EP-A-787 731 et WO-A- 96/08537.

Par « nacres », il faut comprendre des particules colorées de toute forme, irisées ou non, notamment, produites par certains mollusques dans leur coquille ou bien synthétisées et qui présentent un effet de couleur par interférence optique.

Les nacres peuvent être choisies parmi les pigments nacrés, tels que le mica titane recouvert avec un oxyde de fer, le mica titane recouvert avec de l'oxychlorure de bismuth, le mica titane recouvert avec de l'oxyde de chrome, le mica titane recouvert avec un colorant organique, ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

On peut également citer, à titre d'exemple de nacres, le mica naturel recouvert d'oxyde de titane, d'oxyde de fer, de pigment naturel ou d'oxychlorure de bismuth.

La composition cosmétique selon l'invention peut comprendre également des colorants hydrosolubles ou liposolubles. Les colorants liposolubles sont, par exemple, le rouge Soudan, le DC Red 17, le DC Green 6, le β-carotène, l'huile de soja, le brun Soudan, le DC Yellow 11, le DC Violet 2, le DC orange 5 et le jaune quinoléine. Les colorants hydrosolubles sont, par exemple, le jus de betterave et le caramel.

La composition cosmétique selon l'invention peut également contenir au moins un matériau à effet optique spécifique.

Cet effet est différent d'un simple effet de teinte conventionnel, c'est-à-dire unifié et stabilisé tel que produit par les matières colorantes classiques, comme, par exemple, les pigments monochromatiques. Au sens de l'invention, « stabilisé » signifie dénué d'effet de variabilité de la couleur avec l'angle d'observation ou encore en réponse à un changement de température.

Par exemple, ce matériau peut être choisi parmi les particules à reflet métallique, les agents de coloration goniochromatiques, les pigments diffractants, les agents thermochromes, les agents azurants optiques, ainsi que les fibres, notamment, interférentielles. Bien entendu, ces différents matériaux peuvent être associés de manière à procurer la manifestation simultanée de deux effets, voire d'un nouvel effet conforme à l'invention.

Les particules à reflet métallique utilisables dans l'invention sont en particulier choisies parmi :

- les particules d'au moins un métal et/ou d'au moins un dérivé métallique,
- les particules comportant un substrat organique ou minéral, monomatière ou multimatériaux, recouvert au moins partiellement par au moins une couche à reflet métallique comprenant au moins un métal et/ou au moins un dérivé métallique, et
- les mélanges desdites particules.

Parmi les métaux pouvant être présents dans lesdites particules, on peut citer par exemple Ag, Au, Cu, Al, Ni, Sn, Mg, Cr, Mo, Ti, Zr, Pt, Va, Rb, W, Zn, Ge, Te, Se et leurs mélanges ou alliages. Ag, Au, Cu, Al, Zn, Ni, Mo, Cr, et leurs mélanges ou alliages (par exemple, les bronzes et les laitons) sont des métaux préférés.

## CHARGES

Une composition conforme à l'invention peut également comprendre au moins une charge, de nature organique ou minérale.

Par « charge », il faut comprendre les particules incolores ou blanches, solides de toutes formes, qui se présentent sous une forme insoluble et dispersée dans le milieu de la composition. De nature minérale ou organique, elles permettent de conférer à la composition de la douceur, de la matité et de l'uniformité au maquillage.

Les charges utilisées dans les compositions selon la présente invention peuvent être de formes lamellaires, globulaires, sphériques, de fibres ou de toute autre forme intermédiaire entre ces formes définies.

Les charges selon l'invention peuvent être ou non enrobées superficiellement, et, en particulier, elles peuvent être traitées en surface par des silicones, des acides aminés, des dérivés fluorés ou toute autre substance favorisant la dispersion et la compatibilité de la charge dans la composition.

Parmi les charges minérales utilisables dans les compositions selon l'invention, on peut citer le talc, le mica, la silice, le siloxysilicate de triméthyle, le kaolin, la bentone, le carbonate de calcium et l'hydrogéno-carbonate de magnésium, l'hydroxyapatite, le nitrure de bore, les microsphères de silice creuses (Silice Beads de Maprecos), les microcapsules de verre ou de céramique, les charges à base de silice comme l'Aerosil 200, l'Aerosil 300 ; la Sunsphere H-33, la Sunsphere H-51 commercialisés par Asahi Glass ; le Chemicelen commercialisé par Asahi Chemical ; les composites de silice et de dioxyde de titane, comme la série TSG commercialisée par Nippon Sheet Glass, et leurs mélanges.

Parmi les charges organiques utilisables dans les compositions selon l'invention on peut citer les poudres de

polyamide (Nylon® Orgasol de chez Atochem), de poly-b-alanine et polyéthylène, les poudres de polytétrafluoroéthylène (Téflon®), la lauroyl lysine, l'amidon, les poudres de polymères de tétrafluoroéthylène, les microsphères creuses de polymères, telles l'EXPANCEL (NOBEL INDUSTRIE), les savons métalliques dérivés d'acides organiques carboxyliques ayant de 8 à 22 atomes de carbone, de préférence, de 12 à 18 atomes de carbone, par exemple, le stéarate de zinc, de magnésium ou de lithium, le laurate de zinc, le myristate de magnésium, le Polypore® L 200 (Chemdal Corporation), les microbilles de résine de silicone (Tospearl® de Toshiba, par exemple), les poudres de polyuréthane, en particulier, les poudres de polyuréthane réticulé comprenant un copolymère, ledit copolymère comprenant du triméthylol hexyllactone, comme le polymère d'hexaméthylène di-isocyanate/triméthylol hexyllactone, commercialisé sous la dénomination de PLASTIC POWDER D-400® ou PLASTIC POWDER D-800® par la société TOSHIKI, les microcires de Carnauba, telles que celle commercialisée sous la dénomination de MicroCare 350® par la société MICRO POWDERS, les microcires de cire synthétique, telles que celle commercialisée sous la dénomination de MicroEase 114S® par la société MICRO POWDERS, les microcires constituées d'un mélange de cire de Carnauba et de cire de polyéthylène, telles que celles commercialisées sous les dénominations de Micro Care 300® et 310® par la société MICRO POWDERS, les microcires constituées d'un mélange de cire de Carnauba et de cire synthétique, telles que celle commercialisée sous la dénomination Micro Care 325® par la société MICRO POWDERS, les microcires de polyéthylène, telles que celles commercialisées sous les dénominations de Micropoly 200®, 220®, 220L® et 250S® par la société MICRO POWDERS ; et leurs mélanges.

Les charges peuvent être présentes en une teneur allant de 0,1 à 20 % en poids, de préférence de 0,5 à 15% en poids par rapport au poids total de ladite composition.

## ADDITIFS

Une composition cosmétique selon l'invention peut également comprendre en outre tout additif usuellement utilisé dans le domaine concerné, par exemple choisi parmi des gommes, des agents tensioactifs anioniques, cationiques, amphotériques ou non ioniques, des agents tensioacifs silicones, des gommes, des résines, des agents dispersants, des polymères semi-cristallins, des agent antioxydants, des huiles essentielles, des conservateurs, des parfums, des neutralisants, des agents antiseptiques, des agents protecteurs contre les UV, des actifs cosmétiques, telles que des vitamines, des agents hydratants, des émollients, et leurs mélanges.

Il relève des opérations de routine de l'homme de l'art d'ajuster la nature et la quantité des additifs présents dans les compositions conformes à l'invention, de telle sorte que les propriétés cosmétiques et les propriétés de stabilité désirées de celles-ci n'en soient pas affectées.

Une composition selon l'invention peut, notamment, se présenter sous la forme d'une composition de soin ou de maquillage, notamment de maquillage de la peau, des lèvres ou encore des cils.

Selon un mode préféré, la composition selon l'invention est un fond de teint.

La présente invention est présentée plus en détail dans les exemples décrits ci-après qui ne sont proposés qu'à titre d'illustration de l'invention et ne doivent pas être interprétés comme limitant l'invention. Les valeurs sont exprimées en % en poids.

## EXEMPLES :

### Exemple 1: Préparation d'un copolymère de poly (acrylate d'isobornyle / méthacrylate disobornyle / acrylate isobutyle / acide acrylique)

300 g d'isododécane sont introduits dans un réacteur de 1 litre, puis on augmente la température de façon à passer de la température ambiante (25 ˚C) à 90 ˚C en 1 heure.

On ajoute ensuite, à 90 ˚C et en 1 heure, 105 g de méthacrylate d'isobornyle, 105 g d'acrylate d'isobornyle et 1,8 g de 2.5- Bis(2-éthylhexanoylperoxy)-2.5-diméthylhexane (Trigonox® 141 d'Akzo Nobel).

Le mélange est maintenu 1 h30 à 90 ˚C.

On introduit ensuite au mélange précédent, toujours à 90 ˚C et en 30 minutes, 75 g d'acrylate d'isobutyle, 15 g d'acide acrylique et 1,2 g de 2.5-Bis (2-ethylhexanoylperoxy)-2.5-diméthylhexane.

Le mélange est maintenu 3 heures à 90 ˚C, puis l'ensemble est refroidi.

On obtient une solution à 50 % de matière active en copolymère dans l'isododécane.

On obtient un copolymère comprenant une première séquence ou bloc poly(acrylate d'isobornyle/méthacrylate d'isobornyle) ayant une Tg de 128 ˚C, une deuxième séquence poly (acrylate d'isobutyle/acide acrylique) ayant une Tg de -9 ˚C et une séquence intermédiaire qui est un copolymère statistique acrylate d'isobornyle/méthacrylate d'isobornyle/ acrylate d'isobutyle/acide acrylique.

La Tg du copolymère est de 74 ˚C.

Il s'agit de Tg théoriques calculées par la loi de Fox.

**Exemples 2 à 5 : Fonds de teint E/H : influence de la nature de l'huile non volatile**

| | | % massique |
|---|---|---|
| **A1** | PEG-30 Dipolyhydroxy stearate vendu sous la référence ARLACEL P 135 par la Société UNIQEMA | 4,00 |
| | **Huile non volatile** | 1,00 |
| | Mélange n-undécane : n-tridécane, la quantité de n-undécane étant majoritaire dans le mélange* | 12,00 |
| **A2** | Gel de bentone vendu sous la référence Bentone Gel ISD V par la société Elementis | 3,00 |
| **A3** | **Huile non volatile** | 3,00 |
| | Oxyde de fer jaune enrobé de stearoyl glutamate d'aluminium | 1,79 |
| | Oxyde de fer jaune enrobé de stearoyl glutamate 0,54 d'aluminium | |
| | Oxyde de fer noir enrobé de stearoyl glutamate d'aluminium | 0,19 |
| | Dioxyde de titane enrobé de stearoyl glutamate 7,48 d'aluminium | |
| **A4** | Isododécane | 9,40 |
| | Poly (Methacrylate d'isobornyle-co-acrylate d'isobornyle-co-acrylate d'isobutyle-co-acide acrylique) à 50% en matière active dans l'isododécane | 16,00 |
| **A5** | Microsphère de silice vendue sous la référence 700 par la société Miyoshi Kasei | SB 5,50 |
| **A6** | Parfum | 0,30 |
| **B** | Eau déminéralisée | 27,50 |
| | Methyl paraben | 0,20 |
| | PEG 20 | 1,70 |
| | Sulfate de magnésium | 0,70 |
| | Phenoxy éthanol | 0,70 |
| **E** | Ethanol | 5,00 |
| | TOTAL | 100% massique |
| * tel que préparé selon la demande WO2008/155059 | | |

| | **Exemple 2 (Invention)** | **Exemple 3 (Invention)** | **Exemple 4 (comparatif)** | **Exemple 5 (comparatif)** |
|---|---|---|---|---|
| Nature de l'huile non volatile | Néopentanoat e d'isodécyle. | Di capryl ether vendu sous la référence la CETIOL OE par la Société COGNIS. | Isohexadécane | Octyldodécanol |
| $\delta a$ $(J/cm^3)^{1/2}$ | 5,32 | 3,49 | 0 | 7,73 |
| M (g/mol) | 242,4 | 242 | 226,45 | 298,55 |

Mode opératoire

Dans le bécher principal, on introduit les constituants de la phase A1 que l'on chauffe à 50-55°C jusqu'à l'obtention d'un mélange liquide et homogène.

On introduit ensuite la phase A2 à température ambiante et sous agitation (Agitateur de type Moritz : 1000 trs/mn), jusqu'à l'obtention d'un mélange homogène.
La phase A3 est préparée séparément en broyant trois fois à la tricylindre le mélange de pigments et de l'huile non volatile. Cette phase A3 est ajoutée sous agitation, puis on introduit successivement la phase A4 qui a été préparée séparément en diluant le polymère par de l'isododécane, la phase A5 et la phase A6.
La préparation de la phase aqueuse B est réalisée de la manière suivante : on pèse dans un "bécher le méthyl paraben, le sulfate de magnésium et le PEG-20. On ajoute ensuite l'eau préalablement portée à ébullition et dont la température est voisine de 85-90°C, puis on agite à l'aide d'un barreau aimanté jusqu'à dissolution des trois constituants. On laisse la température redescendre à 40°C et on ajoute le phenoxy éthanol.
L'émulsion est réalisée à température ambiante : on verse la phase aqueuse B dans la phase grasse, en augmentant progressivement l'agitation (Moritz) jusqu'à 4500trs/mn. On maintient cette agitation pendant 10mn et on ajoute enfin la phase C (éthanol).
Le produit obtenu est placé sous agitation Rayneri (pâles), et agité pendant 10mn à 10trs/mn.

## Mesure de la matité et de la tenue de la matité

### Principe de la mesure

La matité du visage est mesurée à l'aide de la caméra polarimétrique, qui est un système d'imagerie polarimétrique en noir et blanc, avec laquelle nous acquérons des images en lumière polarisée parallèle (P) et croisée (C). Par analyse de l'image résultant de la soustraction des deux images (P-C), on quantifie la brillance et ceci en mesurant le niveau de gris moyen des 5% de pixels les plus brillants correspondant aux zones de brillance.

### Déroulement du test

Le test se déroule de la manière suivante :

16 femmes arrivent en salle d'attente climatisée (22°C +/- 2°C) 15 mn avant le début du test.

Elles se démaquillent et on réalise avec la caméra polarimétrique une image d'une de leurs joues. Cette image permet de mesurer la brillance à T0 avant maquillage.
Puis on pèse 100 mg de fond de teint dans un verre de montre que l'on applique aux doigts nus sur le demi visage sur lequel on a réalisé la mesure à T0.
Après un temps de séchage de 15 mn, on réalise alors avec la caméra polarimétrique une image de la joue maquillée. Cette image permet de mesurer la brillance juste après maquillage (Timm).
Les modèles retournent alors en salle climatisée pendant 3 h.
On réalise enfin avec la caméra polarimétrique une image de la joue maquillée après 3h d'attente. Cette image permet de mesurer la brillance après 3 h de maquillage (T3h).

### Expression des résultats

On calcule la différence (Timm - T0) qui mesure l'effet du maquillage. Une valeur négative signifie que le maquillage diminue la brillance de la peau et qu'il est donc matifiant.
On calcule ensuite la différence (T3h - Timm) qui mesure la tenue de cet effet. La valeur obtenue doit être la plus faible possible ce qui signifie que la matité du maquillage ne change pas au cours de temps.

| | **Exemple 2 (Invention)** | **Exemple 3 (Invention)** | **Exemple 4 (comparatif)** | **Exemple 5 (comparatif)** |
|---|---|---|---|---|
| Nature de l'huile non volatile | Néopentanoate d'isodécyle. | Di capryl ether vendu sous la référence CETIOL OE par la Société COGNIS. | Isohexadecane | Octyldodecanol |

(suite)

| | Exemple 2 (Invention) | Exemple 3 (Invention) | Exemple 4 (comparatif) | Exemple 5 (comparatif) |
|---|---|---|---|---|
| Matité (Timm-T0) | -3,85 | -3,36 | -5,97 | -5,74 |
| Tenue de la matité (T3h-Timm) | 3,52 | 4,37 | 7,32 | 6,43 |

Ces résultats montrent que les huiles non volatiles de l'invention (Ex.2 et 3) conduisent aux meilleures performances de tenue.

**Revendications**

**1.** Composition cosmétique de soin et/ou de maquillage des matières kératiniques comprenant au moins une phase grasse liquide et au moins :

a) un copolymère éthylénique séquencé (également appelé polymère éthylénique séquencé), contenant au moins une première séquence ayant une température de transition vitreuse (Tg) supérieure ou égale à 40˚C et étant issue en totalité ou en partie de un ou plusieurs premiers monomères, qui sont tels que l'homopolymère préparé à partir de ces monomères a une température de transition vitreuse supérieure ou égale à 40˚C, et au moins une deuxième séquence ayant une température de transition vitreuse inférieure ou égale à 20˚C et étant issue en totalité ou en partie de un ou plusieurs deuxièmes monomères, qui sont tels que l'homopolymère préparé à partir de ces monomères a une température de transition vitreuse inférieure ou égale à 20˚C, ladite première séquence et ladite deuxième séquence étant reliées entre elles par un segment intermédiaire statistique comprenant au moins un desdits premiers monomères constitutifs de la première séquence et au moins un desdits deuxièmes monomères constitutifs de la deuxième séquence, et ledit copolymère séquencé ayant un indice de polydispersité I supérieur à 2,
b) une huile non volatile hydrocarbonée **caractérisée par** un paramètre de solubilité de Hansen δa allant de 2 à 7 (J/cm3)1/2 et une masse moléculaire inférieure ou égale à 300g/mol, et
c) une huile volatile.

**2.** Composition selon la revendication précédente, **caractérisée en ce qu'**elle est sous la forme d'une émulsion, de préférence d'une émulsion eau-dans-huile (E/H).

**3.** Composition selon l'une des revendications précédentes, **caractérisée en ce que** le ou lesdits premiers monomères, qui sont tels que l'homopolymère préparé à partir de ces monomères a une températures de transition vitreuse supérieure ou égale à 40˚C, sont choisis parmi :

- les méthacrylates de formule $CH_2 = C(CH_3)$-COOR,
dans laquelle $R_1$ représente un groupe alkyle non substitué, linéaire ou ramifié, contenant de 1 à 4 atomes de carbone, un groupe cycloalkyle $C_4$ à $C_{12,}$,
- les acrylates de formule $CH_2$ = CH-$COOR_2$
dans laquelle $R_2$ représente un groupe cycloalkyle en $C_4$ à $C_{12}$,
- les (méth)acrylamides de formule :

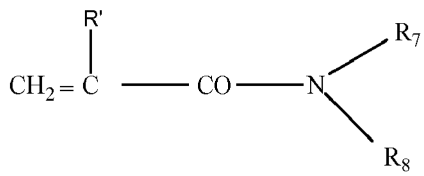

où $R_7$ et $R_8$ identiques ou différents représentent chacun un atome d'hydrogène ou un groupe alkyle en $C_1$ à $C_{12}$ linéaire ou ramifié, ou $R_7$ représente H et $R_8$ représente un groupement 1,1-diméthyl-3-oxobutyl,

et R' désigne H ou méthyle.

et **en ce que** le ou lesdits deuxièmes monomères, qui sont tels que l'homopolymère préparé à partir de ces monomères a une températures de transition vitreuse inférieure ou égale à 20°C, sont choisis parmi :

- les acrylates de formule $CH_2$ = $CHCOOR_3$,

$R_3$ représentant un groupe alkyle non substitué en $C_1$ à $C_{12}$, linéaire ou ramifié, à l'exception du groupe tertiobutyle, dans lequel se trouve(nt) éventuellement intercalé(s) un ou plusieurs hétéroatomes choisis parmi O, N, S,

- les méthacrylates de formule $CH_2$ = $C(CH_3)$-$COOR_4$,

$R_4$ représentant un groupe alkyle non substitué en $C_6$ à $C_{12}$ linéaire ou ramifié, dans lequel se trouve(nt) éventuellement intercalé(s) un ou plusieurs hétéroatomes choisis parmi 0, N et S;

- les esters de vinyle de formule $R_5$-CO-O-CH = $CH_2$

où $R_5$ représente un groupe alkyle en $C_4$ à $C_{12}$ linéaire ou ramifié ;

- les éthers d'alcool vinylique et d'alcool en $C_4$ à $C_{12}$,
- les N-alkyl en $C_4$ à $C_{12}$ acrylamides, tels que le N-octylacrylamide,
- et leurs mélanges.

**4.** Composition la revendication précédente, dans laquelle ledit copolymère séquencé est tel que
la première séquence est obtenue à partir d'au moins un monomère acrylate de formule $CH_2$ = CH-$COOR_2$ dans laquelle $R_2$ représente un groupe cycloalkyle $C_4$ à $C_{12}$ et d'au moins un monomère méthacrylate de formule $CH_2$ = $C(CH_3)$-COOR'$_2$ dans laquelle R'$_2$ représente un groupe cycloalkyle $C_4$ à $C_{12}$, et
la deuxième séquence est obtenue à partir d'au moins un deuxième monomère tel que l'homopolymère obtenu a une température de transition vitreuse inférieure ou égale à 20 °C, et d'un monomère additionnel de type acide acrylique.

**5.** Composition selon la revendication précédente, dans laquelle $R_2$ et R'$_2$ représentent indépendamment ou simultanément un groupe isobornyle.

**6.** Composition selon l'une quelconque des revendications précédentes, dans laquelle ledit copolymère séquencé comprend de 50 à 80 % en poids de méthacrylate/acrylate d'isobornyle, de 10 à 30 % en poids d'acrylate d'isobutyle et de 2 à 10 % en poids d'acide acrylique.

**7.** Composition selon l'une quelconque des revendications précédentes, comprenant de 0,5 à 30% en poids, de préférence de 1 à 20% en poids, préférentiellement encore de 1 à 10% en poids de matière active de copolymère éthylénique séquencé (a) par rapport au poids total de la composition.

**8.** Composition selon l'une des revendications précédentes, dans laquelle l'huile non volatile hydrocarbonée (b) est choisie parmi l'isodecyl néopentanoate, le myristate d'isopropyle, le palmitate d'isopropyle, l'ethyl-2 hexanoate d'ethyl-2 hexyle, l'isononyl isononanoate, l'isononanoate d'isodecyle, le dicapryl carbonate, le dicapryl ether, et leurs mélanges.

**9.** Composition selon la revendication précédente, dans laquelle l'huile non volatile hydrocarbonée (b) est l'isodécyl néopentanoate.

**10.** Composition selon l'une des revendications précédentes, dans laquelle l'huile non volatile hydrocarbonée (b) est présente en une teneur allant de 0,1 à 30% en poids, de préférence de 1 à 20% en poids, et plus préférentiellement de 2 à 10% en poids par rapport au poids total de ladite composition.

**11.** Composition selon l'une quelconque des revendications précédentes, **caractérisée en ce que** l'huile volatile (c) est une huile volatile hydrocarbonée.

**12.** Composition selon l'une quelconque des revendications précédentes, **caractérisée en ce que** l'huile volatile (c) est présente en une teneur allant de 1 à 80% en poids, de préférence de 10 à 60% en poids, et plus préférentiellement de 20 à 50% en poids par rapport au poids total de ladite composition.

**13.** Composition cosmétique selon l'une quelconque des revendications précédentes, **caractérisée en ce qu'**elle comprend en outre au moins une matière colorante.

**14.** Composition selon l'une quelconque des revendications précédentes, **caractérisée en ce qu'**il s'agit d'un fond de teint.

**15.** Procédé cosmétique de soin et/ou de maquillage des matières kératiniques comprenant l'application sur lesdites matières kératiniques, et notamment la peau d'une composition telle que définie dans l'une quelconque des revendications précédentes.

Europäisches Patentamt
European Patent Office
Office européen des brevets

## RAPPORT PARTIEL DE RECHERCHE EUROPEENNE

selon la règle 62a et/ou 63 de la Convention sur le brevet européen. Ce rapport est consideré, aux fins de la procédure ultérieure, comme le rapport de la recherche européenne.

Numéro de la demande
EP 10 17 5376

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | FR 2 860 156 A1 (OREAL [FR]) 1 avril 2005 (2005-04-01) * revendications 1,17,23,43; exemple 1 * | 1,15 | INV. A61K8/31 A61K8/33 A61K8/37 A61K8/81 A61K8/90 A61Q1/00 A61Q19/00 |
| X | EP 1 411 069 A2 (OREAL [FR]) 21 avril 2004 (2004-04-21) * alinéas [0003], [0005], [0013], [0014], [0079] - [0086], [0150], [0190]; exemple 27 * | 1,15 | |
| X | EP 1 518 535 A1 (OREAL [FR]) 30 mars 2005 (2005-03-30) * alinéas [0010], [0011], [0015], [0016], [0125]; revendication 1; exemples 1-3 * | 1,15 | |
| X | EP 1 882 709 A1 (OREAL [FR]) 30 janvier 2008 (2008-01-30) * alinéas [0001], [0002]; revendications 1,12; exemple 1 * | 1,15 | |

DOMAINES TECHNIQUES RECHERCHES (IPC)

A61K
A61Q

### RECHERCHE INCOMPLETE

La division de la recherche estime que la présente demande de brevet, ou une ou plusieurs revendications, ne sont pas conformes aux dispositions de la CBE de façon que seulement une recherche partielle a été établie.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

1

| Lieu de la recherche | Date d'achèvement de la recherche | Examinateur |
|---|---|---|
| Munich | 3 mars 2011 | Yon, Jean-Michel |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04E08)

Europäisches Patentamt
European Patent Office
Office européen des brevets

## RECHERCHE INCOMPLETE
## FEUILLE SUPPLEMENTAIRE C

Numéro de la demande
EP 10 17 5376

Revendications susceptibles de faire l'objet de recherches complètes:
6

Revendications ayant fait l'objet de recherches incomplètes:
1-5, 7-15

Raison pour la limitation de la recherche:

La présente revendication 1 couvre des composés définis seulement par leur fonction souhaitée, ce qui est contraire à l'exigence de clarté, étant donné qu'une définition du type "résultat recherché" ne permet pas de déterminer la portée de la revendication. Le fait que chaque composé puisse être testé ne lève pas cette objection car, mis à part les composés divulgués dans la description (cf.page 4, lignes 3 à 5 ; page 16, lignes 6 à 8 et exemple 1), l'homme du métier ne saurait pas de prime abord si un composé entrerait dans le cadre de la portée revendiquée. Un nombre excessif d'essais serait nécessaire afin de tester aléatoirement les composés. La demande n'est pas conforme aux dispositions de fond au point qu'une recherche significative n'a pu être effectuée au regard de l'ensemble de l'objet revendiqué.
La recherche a par conséquent été limitée aux composés comprenant des monomères acrylate d'isobornyle et méthacrylate d'isobornyle et des monomères acrylate d'isobutyle et acide acrylique.

**ANNEXE AU RAPPORT DE RECHERCHE EUROPEENNE RELATIF A LA DEMANDE DE BREVET EUROPEEN NO.** EP 10 17 5376

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

03-03-2011

| Document brevet cité au rapport de recherche | Date de publication | Membre(s) de la famille de brevet(s) | Date de publication |
|---|---|---|---|
| FR 2860156 A1 | 01-04-2005 | AUCUN | |
| EP 1411069 A2 | 21-04-2004 | AT 486581 T | 15-11-2010 |
| | | BR 0303891 A | 08-09-2004 |
| | | CN 1504488 A | 16-06-2004 |
| | | JP 2004149772 A | 27-05-2004 |
| | | JP 2008069362 A | 27-03-2008 |
| | | KR 20040027429 A | 01-04-2004 |
| | | MX PA03008714 A | 10-09-2004 |
| | | PT 1411069 E | 11-01-2011 |
| | | US 2004120920 A1 | 24-06-2004 |
| | | US 2008014158 A1 | 17-01-2008 |
| EP 1518535 A1 | 30-03-2005 | AT 439118 T | 15-08-2009 |
| | | CN 1636539 A | 13-07-2005 |
| | | CN 101856314 A | 13-10-2010 |
| | | ES 2330219 T3 | 07-12-2009 |
| | | FR 2860143 A1 | 01-04-2005 |
| | | JP 4122324 B2 | 23-07-2008 |
| | | JP 2005104979 A | 21-04-2005 |
| | | KR 20050030589 A | 30-03-2005 |
| | | US 2005106197 A1 | 19-05-2005 |
| EP 1882709 A1 | 30-01-2008 | AT 449801 T | 15-12-2009 |
| | | BR PI0702106 A | 01-04-2008 |
| | | CN 101113193 A | 30-01-2008 |
| | | ES 2335367 T3 | 25-03-2010 |
| | | FR 2904320 A1 | 01-02-2008 |
| | | JP 2008031479 A | 14-02-2008 |
| | | KR 20080011121 A | 31-01-2008 |
| | | US 2008031837 A1 | 07-02-2008 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1411069 A, de L'Oreal
- EP 1518535 A, de L'Oreal
- EP 1411069 A2, de l'Oréal
- WO 2007068371 A1
- EP 542669 A
- EP 787730 A
- EP 787731 A
- WO 9608537 A
- WO 2008155059 A


### Littérature non-brevet citée dans la description


- Polymer Handbook. John Wiley, 1989
- **C. M. HANSEN.** The three dimensionnal solubility parameters. *J. Paint Technol.,* 1967, vol. 39, 105